# FASCICULE DE BREVET EUROPEEN

(11) **EP 2 026 779 B1**
(45) Date de publication et mention de la délivrance du brevet: **26.09.2012**
(21) Numéro de dépôt: 07729341.3
(22) Date de dépôt: 21.05.2007
(51) Int. Cl.: A61K 31/05, A61K 31/57, A61K 31/203, A61K 31/585, A61K 8/63, A61K 8/67, A61K 8/35, A61Q 19/02, A61P 17/00, A61P 17/08

(54) **UTILISATION D'UNE COMPOSITION COMPRENANT UNE ASSOCIATION D'HYDROQUINONE, D'ACÉTONIDE DE FLUOCINOLONE, ET DE TRÉTINOINE, DESTINÉE AU TRAITEMENT DES SIGNES CUTANÉS DU PHOTOVIEILLISSEMENT**
VERWENDUNG EINER ZUSAMMENSETZUNG AUS EINER KOMBINATION VON HYDROCHINON, FLUOCINOLONACETONID UND TRETINOIN ZUR BEHANDLUNG VON ANZEICHEN VON HAUT-LICHTALTERUNG
USE OF A COMPOSITION CONSISTING OF A COMBINATION OF HYDROQUINONE, FLUOCINOLONE ACETONIDE AND TRETINOIN, INTENDED FOR TREATMENT SIGNS OF PHOTOAGING OF THE SKIN

(30) Priorité: 19.05.2006 FR 0604504
(43) Date de publication de la demande: 25.02.2009
(73) Titulaire: Galderma S.A., 6330 Cham (CH)
(72) Inventeur: HEXSEL, Doris Maria, 90520-000 Porto Alegre (BR)
(74) Mandataire: Boulard, Denis
(86) Numéro de dépôt international: PCT/EP2007/054897
(87) Numéro de publication internationale: WO 2007/135132

(56) Documents cités:
- WO-A1-00/40217
- US-A- 3 856 934
- US-A1- 2004 081 668
- TOROK H M ET AL: "HYDROQUINONE 4%, TRETINOIN 0.05%, FLUOCINOLONE ACETONIDE 0.01%: A SAFE AND EFFICACIOUS 12-MONTH TREATMENT FOR MELASMA" CUTIS, EXCERPTA MEDICA, BELLE MEAD,NJ, US, vol. 75, no. 1, janvier 2005 (2005-01), pages 57-62, XP009067213 ISSN: 0011-4162
- COOK-BOLDEN F ET AL: "THE USE OF A TRIPLE-DRUG COMBINATION PRODUCT AND PROCEDURES FOR THE TREATMENT OF HYPERPIGMENTARY DISORDERS" COSMETIC DERMATOLOGY, KNOLLS PUB. GROUP, CEDAR KNOLLS, NJ, US, vol. 18, no. 8, août 2005 (2005-08), pages 589-594, XP009066078 ISSN: 1041-3766
- ROLLMAN O ET AL: "CUTANEOUS VITAMIN A LEVELS IN SEBORRHEIC KERATOSIS ACTINIC KERATOSIS AND BASAL CELL CARCINOMA" ARCHIVES OF DERMATOLOGICAL RESEARCH, vol. 270, no. 2, 1981, pages 193-196, XP009077650 ISSN: 0340-3696

## Description

La présente invention concerne une composition dermatologique comprenant une association d'hydroquinone, d'acétonide de fluocinolone, et de trétinoine, destinée au traitement des signes cutanés du photovieillissement.

La peau humaine est constituée de deux compartiments, à savoir un compartiment profond, le derme, et un compartiment superficiel, l'épiderme.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale. On y trouve aussi des leucocytes, des mastocytes ou encore des macrophages tissulaires. Il contient également des vaisseaux sanguins et des fibres nerveuses.

L'épiderme est en contact avec l'environnement extérieur. Il est composé de kératinocytes, de mélanocytes et des cellules de Langerhans. Son rôle consiste à protéger l'organisme de la déshydratation et des agressions extérieures, qu'elles soient chimiques, mécaniques, physiques ou infectieuses. L'accumulation de ces agressions tout au long de la vie, couplée à l'influence génétique, conduit au vieillissement de la peau.

Le vieillissement cutané comprend deux phénomènes distincts : le vieillissement intrinsèque, conséquence du passage du temps, et le photovieillissement, conséquence de l'exposition solaire. Le vieillissement intrinsèque de la peau se traduit par un aspect finement ridé, laxe, sec avec éventuellement présence de tumeurs bénignes. Le photovieillissement cutané lui, s'observe au niveau des zones habituellement exposées au soleil, comme la face, le dos des mains et des avant-bras, et le décolleté (Ann Dermatol Venereol (2005) : 132 : 362-7).

La sévérité et l'aspect clinique du photovieillissement cutané sont déterminés par l'exposition cumulée aux UV, et par la capacité individuelle de l'individu à répondre à cette exposition. En effet, les personnes de phototypes I et II, présentent un ensemble de signes légèrement différents des personnes de phototype III à V, à peau mate.

Le photovieillissement peut être caractérisé par différents signes cutanés, tels que la rugosité cutanée, les kératoses actiniques, l'élastose solaire, les rides (fines et profondes), les télangiectasies, l'hyperpigmentation diffuse ou encore d'autres désordres pigmentaires tels que les lentigines solaires et les éphélides.

Ce phénomène est clairement à distinguer du mélasma. En effet, le mélasma, appelé également chloasma ou masque de grossesse, est une forme acquise d'hyperpigmentation se manifestant principalement sur le visage. Il peut résulter d'une grossesse, de l'utilisation de contraceptif oral, de la ménopause, ou peut se manifester de novo sans raison apparente. Il est exacerbé par une exposition aux UV (« Hyperpigmentation disorders », Goodheart, Women's Health in Primary Care, vol.2, No.12/December 1999).

Il consiste en un brunissement par taches asymptomatique de la peau du visage. Les lésions sont foncées à marron, les macules hyperpigmentées peuvent coalescer en zones symétriques bien démarquées. On les trouve principalement sur les joues, le front, le nez, sur la lèvre supérieure ou sur le menton. La zone cutanée foncée peut spontanément disparaître après la grossesse, l'arrêt du contraceptif oral ou l'absence d'exposition au soleil.

Le mélasma est à distinguer des lentigines solaires, des éphélides et de l'hyperpigmentation post-inflammatoire (« Hyperpigmentation disorders », Goodheart, Women's Health in Primary Care, vol.2, No.12/December 1999).

Aussi le photovieillissement présente des signes cutanés spécifiques, et ne peut être confondu avec le mélasma.

Parmi ces signes cutanés, les lentigines sont de petites macules que l'on retrouve surtout au dos des mains et des bras, sur le décolleté ou encore au niveau de la face. Ces taches sont plus foncées que les éphélides, et persistent en hiver. Elles sont dues non seulement à une adaptation des cellules qui fabriquent une mélanine brune ou rouge, mais aussi à une multiplication du nombre des mélanocytes. La couleur de ces taches est uniforme, et elles apparaissent avec l'âge sur les zones exposées au soleil, comme le visage et le dos des mains. Ce désordre est également appelé lentigo solaire.

L'hyperpigmentation diffuse, appelée « mottled hyperpigmentation » en anglais, se traduit par une irrégularité diffuse de la coloration cutanée.

Des signes de kératoses actiniques sont aussi retrouvés sur la peau photovieillie ; ces lésions, asymptomatiques la plupart du temps, peuvent être aussi considérées comme des lésions précancéreuses. Ce sont des épaississements localisés de la peau, hyperkératosiques et présentant des squames. Si le patient cherche à arracher ces squames, un léger saignement survient.

Par élastose solaire, on entend le relâchement de la peau, qui perd de son élasticité, en raison de l'altération et de la casse des fibres d'élastine synthétisées par les fibroblastes du derme.

Par télangiectasie, on entend une dilatation permanente des petits vaisseaux, capillaires, artérioles ou veinules, qui dessine de petites lignes rouges sous la peau.

Les individus de phototype I et II sont plus touchés par les lentigines et les ridules, tandis que les individus de phototype III à V sont également touchés par les lentigines accompagnées de rides profondes, d'un aspect nodulaire et grossier de la peau correspondant à une élastose.

La prévention du photovieillissement est devenue depuis quelques années un véritable enjeu de santé publique, du fait des conséquences dramatiques qu'il peut entraîner, i.e l'augmentation de la prévalence des cancers de la peau (mélanomes).
Mais aujourd'hui, au-delà de la prévention, il existe un réel besoin de traitement des signes cutanés du photovieillissement.
Un seul agent topique a démontré son efficacité dans la correction des signes du photovieillissement ; il s'agit de la trétinoine. La demande WO00/40217 décrit que la l'utilisation de trétinoïne peut être efficace pour le traitement des signes cutanés du photoviellissement.

Elle améliore l'apparence de la peau photovieillie, avec surtout une amélioration de la rugosité de la peau, des ridules, de la pigmentation. Mais le traitement à la trétinoine est long, environ 6 mois, et doit être chronique, ce qui ne facilite pas l'observance du traitement par les patients.

D'autres traitements existent à l'heure d'aujourd'hui, comme le traitement à l'aide d'alpha-hydroxyacides, ou encore de béta-hydroxyacides. Mais ces derniers ne semblent pas assez efficaces, notamment dans le traitement des tâches pigmentaires.
Le traitement à l'aide de peeling chimique est certes beaucoup plus efficace, mais il ne peut être considéré comme dénué de risques. En effet, un phénomène de rebond pigmentaire peut être observé à la suite d'un traitement par peeling chimique.

D'autres techniques, telles que la dermabrasion ou encore diverses sources de lumière (dont le laser au CO₂) sont utilisées elles aussi, mais elles sont tout aussi lourdes et posent les mêmes inconvénients que le peeling chimique.

En conséquence, il existe donc un réel besoin d'un traitement efficace et sans risque des symptômes du photovieillissement, en particulier les tâches hyperpigmentaires induites par l'exposition aux rayons ultraviolets.

De façon surprenante, la Demanderesse a découvert que l'association d'hydroquinone, d'acétonide de fluocinolone et de trétinoïne dans une composition pharmaceutique permet de traiter les signes cutanés du photovieillissement, en particulier les tâches hyperpigmentaires.

La présente invention se rapporte donc à l'utilisation d'une association d'acétonide de fluocinolone, d'hydroquinone et de trétinoïne, pour la préparation d'un médicament destiné à traiter les signes cutanés du photovieillissement.

Selon l'invention, par signes cutanés du photovieillissement, on entend les désordres cutanés hyperpigmentaires et non pigmentaires induits par les rayonnements UV.
Parmi les désordres cutanés pigmentaires induits par les rayonnements UV, on entend la kératose actinique, l'hyperpigmentation diffuse, le lentigo solaire (ou lentigo sénile), les éphélides, ou encore les verrues séborrhériques pigmentées planes.

Par verrue séborrhéique pigmentée plane, on entend une lésion (sans gravité) colorée et en relief apparaissant sur le dos et le thorax principalement, et dont la surface ressemble à celle d'un chou-fleur.

Parmi les désordres cutanés non pigmentaires induits par les rayonnements UV, on entend l'élastose solaire, les rides et ridules, et la rugosité cutanée.

Préférentiellement, le médicament est destiné à traiter les désordres cutanés hyperpigmentaires.

Avantageusement, le médicament selon la présente invention est destiné à une application topique.

Le médicament selon la présente invention comprend en outre un milieu physiologiquement acceptable, c'est-à-dire qui est compatible avec la peau, et peut constituer une composition dermatologique.

L'hydroquinone est un agent dépigmentant connu. Il est préparé par réduction de la *p-*benzoquinone avec du bisulfite de sodium. Le nom chimique de l'hydroquinone est le 1,4-benzènediol
De façon avantageuse, l'hydroquinone est présente dans le médicament à une concentration comprise entre 1 et 10% en poids, avantageusement entre 2 et 7 %, et plus avantageusement à une concentration de 4% en poids, par rapport au poids total du médicament.

La trétinoïne est un acide all-trans rétinoïque formé par l'oxydation du groupe aldéhyde du rétinène en groupe carboxyle. Le nom chimique de la trétinoïne est l'acide (*all-E*)-3,7-diméthyl-9-(2,6,6-triméthyl-1-cyclohexèn-1-yl)-2,4,6,8-nonatétraénoique. Il est très réactif à la lumière et à l'humidité. Son action sur la différenciation et prolifération kératinocytaire, la synthèse de collagène et son activité dépigmentante sont intéressante dans le traitement du photovieillissement.
Dans un mode de réalisation particulier, la trétinoïne est présente dans le médicament selon la présente invention à une concentration comprise entre 0,025 et 2% en poids, avantageusement entre 0.025% et 1% en poids, encore plus avantageusement d'environ 0,05% en poids, par rapport au poids total du médicament.

L'acétonide de fluocinolone est un corticostéroïde synthétique fluoré destiné à une utilisation dermatologique topique, et il est utilisé en tant qu'anti-inflammatoire. Son nom chimique est le (6,11,16)-6,9-difluoro-11,21-dihydroxy-16,17-[(1-méthyléthylidène)bis(oxy)]-pregna-1,-4-diène-3,20-dione. Il s'agit d'une poudre cristalline blanche qui est sans odeur et stable à la lumière.
Dans un mode de réalisation particulier, l'acétonide de fluocinolone est présent dans le médicament selon la présente invention à une concentration comprise entre 0,005 et 0,1% en poids, avantageusement entre 0,005 et 0,05% en poids, encore plus avantageusement d'environ 0,01 % en poids, par rapport au poids total du médicament.

Avantageusement, le médicament selon la présente invention contient du metabisulfite de sodium pour prévenir l'oxydation de l'hydroquinone.

Des composants additionnels peuvent être présents dans le médicament selon la présente invention en une quantité comprise entre 0,001 et 20 % en poids par rapport au poids total du médicament.

Le médicament selon la présente invention peut comprendre une grande variété de composants additionnels, en particulier il peut s'agir d'absorbants, d'abrasifs, d'agents anti-acné, d'agents anti-mousse, d'agents anti-microbiens, d'anti-oxydants, de liants, d'additifs biologiques, d'agents tampons, d'agents chélatants, de colorants, d'astringents cosmétiques, de biocides cosmétiques, d'analgésiques externes, d'agents formateur de films, de composants parfumés, d'agents opacifiants, de plastifiants, de conservateurs, d'autres agents dépigmentants, d'agents émollients, d'agents protecteurs de la peau, de solvants, d'agents solubilisants, d'agents surfactants, d'agents absorbant les rayonnements ultraviolets, d'agents écrans solaires, d'agents augmentant la viscosité (aqueux ou non aqueux), d'humectants, de séquestrants, etc...

L'homme du métier sera évidemment attentif à choisir les composés additionnels possibles et/ou leur quantité de façon à ce que les propriétés avantageuses du médicament selon la présente invention ne soient pas totalement ou pas substantiellement diminuées par l'ajout envisagé.

Le médicament selon la présente invention peut être fourni sous toutes les formes galéniques normalement utilisées dans le domaine de la dermatologie.

Préférentiellement, la composition selon la présente invention, est sous forme de crème. Par crème, on entend une préparation pour application topique à base d'eau. Elle correspond à une émulsion, i.e. comprend au moins une phase lipophile et au moins une phase hydrophile.

Les crèmes peuvent être avantageusement préparées comme indiqué dans la demande de brevet WO 2004/037201, par un procédé comprenant les étapes de :
a) mélange des composés hydrophiles avec l'eau pour former une phase aqueuse ou hydrophile ;
b) mélange des composés hydrophobes pour former une phase hydrophobe ;
c) mélange des phases hydrophobes et hydrophiles pour former un mélange biphasique et,
d) addition d'un émulsifiant à la phase biphasique pour former une émulsion.

De plus, elles peuvent contenir d'autres ingrédients habituels des crèmes et être fabriquées de façon bien connue de l'homme du métier.
De manière avantageuse, le médicament selon la présente invention contient au moins un ingrédient inactif choisi parmi l'hydroxytoluène butylé, l'alcool cétylique, l'acide citrique, la glycérine, le stéarate de glycéryle, le silicate de magnésium et d'aluminium, le méthyl gluceth-10, le méthyl parabène, le stéarate de PEG-100, le propylparabène, l'eau purifiée, le métabisulfite de sodium, l'acide stéarique et l'alcool stéarylique.

Avantageusement, le médicament selon la présente invention est la crème Tri-Luma® vendue par la société Galderma, telle que présentée dans l'exemple 1.

### Exemple 1 : composition de la crème Tri-Luma®

La crème contient, en pourcentage en poids par rapport au poids total :

| | |
|---|---|
| - silicate de magnésium et d'aluminium | 3,00% |
| - hydroxytoluène butylé | 0,04% |
| - alcool cétylique | 4,00% |
| - acide stéarique | 3,00% |
| - alcool stéarylique | 4,00% |
| - methylparaben | 0,18% |
| - propylparaben | 0,02% |
| - Arlacel® 165 [stéarate de glycérol et monostéarate de glycerol stéarate de PEG-100] | 3,50% |
| - méthyl gluceth-10 | 5,00% |
| - glycérine | 4,00% |
| - trétinoïne | 0,05% |
| - acétonide de fluocinolone | 0,01 % |
| - acide citrique | 0,05% |
| - hydroquinone | 4,00% |
| - métabisulfite de sodium | 0,20% |
| - eau purifiée | 68,95% |

### Exemple 2 : étude d'efficacité de Tri-Luma® sur différents signes cutanés du photovieillissement

60 personnes âgées de 40 à 82 ans ont été randomisée en deux groupes de 30 personnes et ont été traitées sur le visage, respectivement avec le produit Triluma et le second groupe avec de la trétinoine seule à 0.025% (Vitanol A) pendant 8 semaines de traitement.

Différents critères ont été mesurés via un score de sévérité (de *Absent à Sévère* ou *Très Sévère*) au temps T0 avant traitement et à la fin du traitement : Score d'hyperpigmentation diffuse, de sévérité du photovieillissement et de sévérité des rides fines.

L'amélioration ou l'aggravation par rapport à l'état initial avant traitement (T0) a été mesurée pour chaque sujet par la différence *Après - Avant* traitement (T8-T0). Une différence de score négative signifie donc une amélioration (inversement pour une différence positive).

Le tableau ci-dessous présente, pour chaque critère mesuré, la distribution des sujets dans chaque catégorie de différence de score, ainsi que l'analyse de la comparaison entre les traitements.

| Hyperpigmentation diffuse faciale | | Tri_Luma | Vitanol_A | p-value |
|---|---|---|---|---|
| Visite à T 8 semaines | Nombre de sujets = 30 | 100.0% | 100.0% | |
| | 0 | 36.7% | 43.3% | |
| | -1 | 33.3% | 50.0% | |
| | -2 | 30.0% | 6.7% | |
| | Lsmean±Stderr | -0.9±0.1 | -0.6±0.1 | 0.091 |
| | Mean±sd | -0.9±0.8 | -0.6±0.6 | |
| Mesure de la sévérité Photovieillissement | | Tri_Luma | Vitanol_A | p-value |
| Visite à T 8 semaines | Nombre de sujets = 30 | 100.0% | 100.0% | |
| | 0 | 60.0% | 60.0% | |
| | -1 | 23.3% | 36.7% | |
| | -2 | 16.7% | 3.3% | |
| | Lsmean±Stderr | -0.6±0.1 | -0.4±0.1 | 0.502 |
| | Mean±sd | -0.6±0.8 | -0.4±0.6 | |
| Mesure sévérité rides fines | | Tri_Luma | Vitanol_A | p-value |
| Visite à T 8 semaines | Nombre de sujets = 30 | 100.0% | 100.0% | |
| | 1 | 3.3% | 3.3% | |
| | 0 | 66.7% | 70.0% | |
| | -1 | 26.7% | 26.7% | |
| | -2 | 3.3% | - | |
| | Lsmean±Stderr | -0.3±0.1 | -0.3±0.1 | 0.968 |
| | Mean±sd | -0.3±0.6 | -0.2±0.5 | |

Ces résultats montrent que le produit Triluma a une activité sur les signes liés au photovieillissement et plus particulièrement sur l'hyperpigmentation diffuse et les rides. Ce produit est beaucoup plus efficace dans le traitement des signes cutanés du photovieillissement que la trétinoine seule.

## Revendications

1. Utilisation d'une association d'acétonide de fluocinolone, d'hydroquinone et de trétinoïne pour la préparation d'un médicament destiné à traiter les signes cutanés du photovieillissement **caractérisée en ce que** les signes cutanés du photovieillissement sont choisis parmi la kératose actinique, l'hyperpigmentation diffuse, le lentigo solaire, les verrues séborrhériques pigmentées planes, les rides et ridules, l'élastose solaire, la rugosité cutanée, et les éphélides.

2. Utilisation selon l'une des revendications précédentes, **caractérisée en ce que** les signes cutanés du photovieillissement sont choisis parmi la kératose actinique, l'hyperpigmentation diffuse, le lentigo solaire et les verrues séborrhériques pigmentées planes.

3. Utilisation selon l'une quelconque des revendications 1 à 2, **caractérisée en ce que** le médicament est sous forme de composition adaptée à une application topique.

4. Utilisation selon l'une quelconque des revendications 1 à 3, **caractérisée en ce que** l'hydroquinone est présente à une concentration comprise entre 1 et 10% en poids par rapport au poids total du médicament.

5. Utilisation selon la revendication 4, **caractérisée en ce que** l'hydroquinone est présente à une concentration comprise entre 2 et 7 % en poids par rapport au poids total du médicament.

6. Utilisation selon la revendication 4 ou 5, **caractérisée en ce que** l'hydroquinone est présente à une concentration de 4% en poids par rapport au poids total du médicament.

7. Utilisation selon l'une quelconque des revendications 1 à 6, **caractérisée en ce que** la trétinoïne est présente à une concentration comprise entre 0,025 et 2% en poids par rapport au poids total.

8. Utilisation selon la revendication 7, **caractérisée en ce que** la trétinoïne est présente à une concentration comprise entre 0,025% et 1% en poids par rapport au poids total.

9. Utilisation selon la revendication 7 ou 8, **caractérisée en ce que** la trétinoïne est présente à une concentration d'environ 0,05% en poids, par rapport au poids total.

10. Utilisation selon l'une quelconque des revendications 1 à 9, **caractérisée en ce que** l'acétonide de fluocinolone est présent à une concentration comprise entre 0,005 et 0, 1 % en poids par rapport au poids total.

11. Utilisation selon la revendication 10, **caractérisée en ce que** l'acétonide de fluocinolone est présent à une concentration comprise entre 0,005 et 0,05% en poids, par rapport au poids total.

12. Utilisation selon la revendication 10 ou 11, **caractérisée en ce que** l'acétonide de fluocinolone est présent à une concentration d'environ 0,01 % en poids, par rapport au poids total.

13. Utilisation selon l'une quelconque des revendications 1 à 12, **caractérisée en ce que** le médicament est sous forme de crème.

14. Utilisation selon l'une quelconque des revendications 1 à 13, **caractérisée en ce que** le médicament est une composition contenant, en pourcentage en poids par rapport au poids total :
| | |
|---|---|
| - silicate de magnésium et d'aluminium | 3,00% |
| - hydroxytoluène butylé | 0,04% |
| - alcool cétylique | 4,00% |
| - acide stéarique | 3,00% |
| - alcool stéarylique | 4,00% |
| - methylparaben | 0,18% |
| - propylparaben | 0,02% |
| - Arlacel® 165 [stéarate de glycérol et monostéarate de glycerol stéarate de PEG-100] | 3,50% |
| - méthyl gluceth-10 | 5,00% |
| - glycérine | 4,00% |
| - trétinoïne | 0,05% |
| - acétonide de fluocinolone | 0,01% |
| - acide citrique | 0,05% |
| | |
|---|---|
| - hydroquinone | 4,00% |
| - métabisuifite de sodium | 0,20% |
| - eau purifiée | 68,95% |

## Claims

1. Use of a combination of fluocinolone acetonide, hydroquinone and tretinoin for the preparation of a medicament for treating the signs of photo-ageing of the skin, **characterized in that** the signs of photo-ageing of the skin are chosen from actinic keratosis, mottled hyperpigmentation, solar lentigo, flat pigmented seborrhoeic warts, wrinkles and fine lines, solar elastosis, skin roughness, and freckles.

2. Use according to the preceding claim, **characterized in that** the signs of photo-ageing of the skin are chosen from actinic keratosis, mottled hyperpigmentation, solar lentigo and flat pigmented seborrhoeic warts.

3. Use according to either of Claims 1 and 2, **characterized in that** the medicament is in a composition form suitable for topical application.

4. Use according to any one of Claims 1 to 3, **characterized in that** hydroquinone is present at a concentration of between 1% and 10% by weight relative to the total weight of the medicament.

5. Use according to Claim 4, **characterized in that** hydroquinone is present at a concentration of between 2% and 7% by weight relative to the total weight of the medicament.

6. Use according to Claim 4 or 5, **characterized in that** hydroquinone is present at a concentration of 4% by weight relative to the total weight of the medicament.

7. Use according to any one of Claims 1 to 6, **characterized in that** tretinoin is present at a concentration of between 0.025% and 2% by weight relative to the total weight.

8. Use according to Claim 7, **characterized in that** tretinoin is present at a concentration of between 0.025% and 1% by weight relative to the total weight.

9. Use according to Claim 7 or 8, **characterized in that** tretinoin is present at a concentration of about 0.05% by weight relative to the total weight.

10. Use according to any one of Claims 1 to 9, **characterized in that** fluocinolone acetonide is present at a concentration of between 0.005% and 0.1% by weight relative to the total weight.

11. Use according to Claim 10, **characterized in that** fluocinolone acetonide is present at a concentration of between 0.005% and 0.05% by weight relative to the total weight.

12. Use according to Claim 10 or 11, **characterized in that** fluocinolone acetonide is present at a concentration of about 0.01% by weight relative to the total weight.

13. Use according to any one of Claims 1 to 12, **characterized in that** the medicament is in the form of a cream.

14. Use according to any one of Claims 1 to 13, **characterized in that** the medicament is a composition containing, in weight percentage relative to the total weight:
| | |
|---|---|
| - magnesium aluminium silicate | 3.00% |
| - butylated hydroxytoluene | 0.04% |
| - cetyl alcohol | 4.00% |
| - stearic acid | 3.00% |
| - stearyl alcohol | 4.00% |
| - methyl paraben | 0.18% |
| - propyl paraben | 0.02% |
| - Arlacel^{®} 165 (glyceryl stearate and glyceryl monostearate PEG-100 stearate] | 3.50% |
| - methyl gluceth-10 | 5.00% |
| - glycerol | 4.00% |
| - tretinoin | 0.05% |
| - fluocinolone acetonide | 0.01% |
| - citric acid | 0.05% |
| - hydroquinone | 4.00% |
| - sodium metabisulfite | 0.20% |
| - purified water | 68.95% |

## Patentansprüche

1. Verwendung einer Kombination von Fluocinolonacetonid, Hydrochinon und Tretinoin für die Herstellung eines Arzneimittels zur Behandlung von Anzeichen von Hautlichtalterung, **dadurch gekennzeichnet, dass** die Anzeichen von Hautlichtalterung aus der Reihe Aktinkeratose, diffuse Hyperpigmentierung, Sonnenflecken, planare pigmentierte seborrhoische Warzen, Falten und Fältchen, solare Elastose, Rauheit der Haut und Sommersprossen ausgewählt sind.

2. Verwendung nach einem der vorhergehenden Ansprüche, **dadurch gekennzeichnet, dass** die Anzeichen von Hautlichtalterung aus der Reihe Aktinkeratose, diffuse Hyperpigmentierung, Sonnenflecken und planare pigmentierte seborrhoische Warzen ausgewählt sind.

3. Verwendung nach einem der Ansprüche 1 bis 2, **dadurch gekennzeichnet, dass** das Arzneimittel in Form einer an die topische Applikation angepassten Zusammensetzung vorliegt.

4. Verwendung nach einem der Ansprüche 1 bis 3, **dadurch gekennzeichnet, dass** das Hydrochinon in einer Konzentration zwischen 1 und 10 Gew.-% in Bezug auf das Gesamtgewicht des Arzneimittels vorliegt.

5. Verwendung nach Anspruch 4, **dadurch gekennzeichnet, dass** das Hydrochinon in einer Konzentration zwischen 2 und 7 Gew.-% in Bezug auf das Gesamtgewicht des Arzneimittels vorliegt.

6. Verwendung nach Anspruch 4 oder 5, **dadurch gekennzeichnet, dass** das Hydrochinon in einer Konzentration von 4 Gew.-% in Bezug auf das Gesamtgewicht des Arzneimittels vorliegt.

7. Verwendung nach einem der Ansprüche 1 bis 6, **dadurch gekennzeichnet, dass** das Tretinoin in einer Konzentration zwischen 0,025 und 2 Gew.-% in Bezug auf das Gesamtgewicht vorliegt.

8. Verwendung nach Anspruch 7, **dadurch gekennzeichnet, dass** das Tretinoin in einer Konzentration zwischen 0,025 und 1 Gew.-% in Bezug auf das Gesamtgewicht vorliegt.

9. Verwendung nach Anspruch 7 oder 8, **dadurch gekennzeichnet, dass** das Tretinoin in einer Konzentration von ungefähr 0,05 Gew.-% in Bezug auf das Gesamtgewicht vorliegt.

10. Verwendung nach einem der Ansprüche 1 bis 9, **dadurch gekennzeichnet, dass** das Fluocinolonacetonid in einer Konzentration zwischen 0,005 und 0,1 Gew.-% in Bezug auf das Gesamtgewicht vorliegt.

11. Verwendung nach Anspruch 10, **dadurch gekennzeichnet, dass** das Fluocinolonacetonid in einer Konzentration zwischen 0,005 und 0,05 Gew.-% in Bezug auf das Gesamtgewicht vorliegt.

12. Verwendung nach Anspruch 10 oder 11, **dadurch gekennzeichnet, dass** das Fluocinolonacetonid in einer Konzentration von ungefähr 0,01 Gew.-% in Bezug auf das Gesamtgewicht vorliegt.

13. Verwendung nach einem der Ansprüche 1 bis 12, **dadurch gekennzeichnet, dass** das Arzneimittel in Form einer Creme vorliegt.

14. Verwendung nach einem der Ansprüche 1 bis 13, **dadurch gekennzeichnet, dass** es sich bei dem Arzneimittel um eine Zusammensetzung handelt, die in Gewichtsprozent in Bezug auf das Gesamtgewicht Folgendes enthält:
| | |
|---|---|
| - Magnesium- und Aluminiumsilikat | 3,00% |
| - Butylhydroxytoluol | 0,04% |
| - Cetylalkohol | 4,00% |
| - Stearinsäure | 3,00% |
| - Stearylalkohol | 4,00% |
| - Methylparaben | 0,18% |
| - Propylparaben | 0,02% |
| - Arlacel® 165 [Glyzerylstearat und Glyzerylmonostearat PEG-100-Stearat] | 3,50% |
| Methyl Gluceth-10 | 5,00% |
| - Glyzerin | 4,00% |
| - Tretinoin | 0,05% |
| - Fluocinolonacetonid | 0,01% |
| - Citronensäure | 0,05% |
| - Hydrochinon | 4,00% |
| - Natriummetabisulfit | 0,20% |
| - gereinigtes Wasser | 68,95% |
